Europäisches Patentamt

European Patent Office

Office européen des brevets

(19)

(11) Publication number: 0 392 605 A1

# EUROPEAN PATENT APPLICATION

(21) Application number: 90200836.6

(22) Date of filing: 06.04.90

(51) Int. Cl.5: C12N 15/16, C12P 21/02, C12N 15/70

(30) Priority: 13.04.89 US 337592

(43) Date of publication of application:
17.10.90 Bulletin 90/42

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Linemeyer, David L.
526 Clark Street
Westfield, NJ 07090(US)
Inventor: Thomas, Kenneth A., Jr.
245 Washington Avenue
Chatham Burough, NJ 07928(US)

(74) Representative: Hesketh, Alan, Dr. et al
European Patent Department Merck & Co.,
Inc. Terlings Park Eastwick Road
Harlow Essex, CM20 2QR(GB)

(54) Method of enhancing acidic fibroblast growth factor expression.

(57) A unique double cistron expression vector for the enhanced expression of recombinant human acidic fibroblast growth factor (r-haFGF) is disclosed. The double cistron expression vector results in about a 5 to 13-fold increase in the expression of r-haFGF.

EP 0 392 605 A1

## A METHOD OF ENHANCING ACIDIC FIBROBLAST GROWTH FACTOR EXPRESSION

BRIEF DESCRIPTION OF THE DRAWINGS

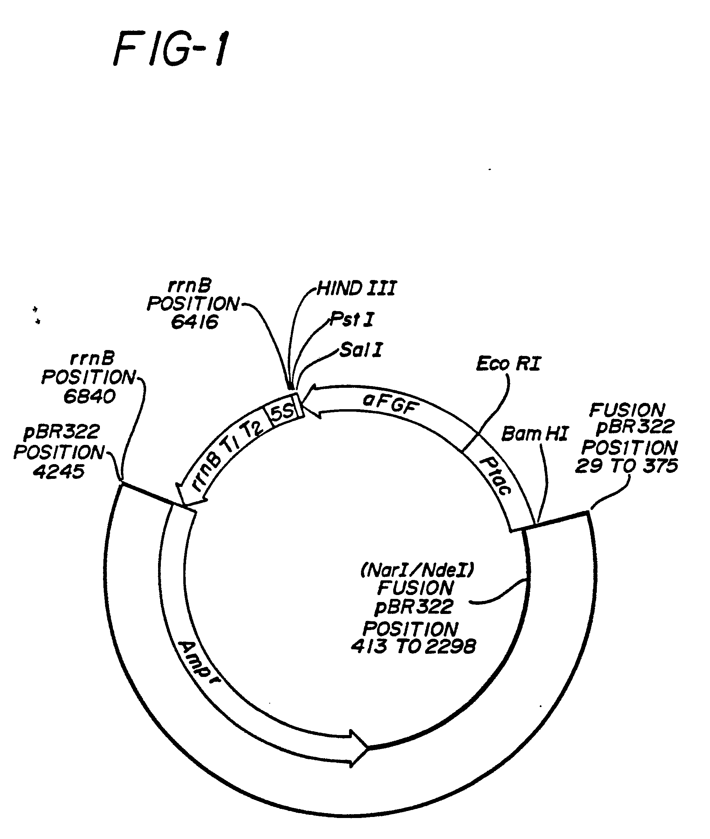

Figure 1 is a diagram of the pKK223-3 plasmid containing a gene for haFGF.

BACKGROUND OF THE INVENTION

Recombinant human acidic fibroblast growth factor (r-haFGF) and methods of expressing the growth factor in Escherichia coli are known. See Linemeyer et al., Bio/Tech. 5: 969-965 (1987) and European Patent Application, Publication No, 259,953. The level of expression is adequate for the isolation and characterization of experimental quantities of r-haFGF but is not adequate for commerical production of this wound healing protein.

High level expression of cloned eucaryotic proteins in E. coli usually involves the incorporation of the gene into a multicopy expression system which will include a strong promoter and result in efficient translation of the message. The level of expression with multicopy expression systems, strong promoters and efficient ribosome binding sites varies widely for different eucaryotic genes, Schoner et al., Proc. Natl. Acad. Sci. USA 83: 8506-8510 (1986). Schoner et al. evaluated a two-cistron expression system for the enhanced expression of bovine growth hormone in E. coli and concluded that the first cistron should be relatively short, contain a Shine-Dalgarno sequence upstream of the stop codon all of which are upstream of the cistron which encodes the desired protein.

OBJECTS OF THE INVENTION

It is accordingly, an object of the present invention to provide an improved method for the production of r-haFGF. Another object is to provide a double cistron expression system for the production of r-haFGF. A further object is to provide a specific double cistron expression system for the production of r-haFGF which will result in about a 15-fold increase in r-haFGF expression.

SUMMARY OF THE INVENTION

A unique double cistron expression vector for the enhanced expression of recombinant human acidic fibroblast growth factor (r-haFGF) is disclosed. The double cistron expression vector results in about a 5 to 13-fold increase in the expression of r-haFGF.

DETAILED DESCRIPTION OF THE INVENTION

Human acidic fibroblast growth factor exists in primarily three microheterogeneous forms. The amino acid and nucleotide base sequences of the longest, or 154 amino acid form, have been described by Jaye et al., Science 233:541-545 (1986). The shorter forms containing either 140 amino acid residues or 139 amino acid residues were described by Gimenez-Gallego et al., Biochem. Biophys. Res. Comm. 138: 611-617 (1986). A synthetic gene for the shorter forms of aFGF has been described by Linemeyer et al., European Patent Application, Publication No. 259,953. The 139 amino acid form is equivalent to the 140 amino acid form with the amino terminal phenylalanine removed. The amino terminal asparagine residue may be deamidated to aspartic acid in the 139 amino acid form of haFGF.

The present invention is contemplated to include genes, either native or synthetic, for all microheterogeneous forms of human aFGF. Native gene is defined herein as a nucleotide sequence of DNA isolated from a human cell capable of producing aFGF. A synthetic gene is defined herein as a nucleotide sequence that has been chemically synthesized and includes the native nucleotide and other nucleotide sequences that will encode for the proper amino acid sequence of haFGF. It is further intended that this invention include nucleotide sequences which may be shorter than the conventional microheterogeneous forms but which encode proteins that have haFGF activity.

Enhanced expression of the haFGF genes is accomplished by designing an expression vector which contains a short DNA sequence inserted upstream from the haFGF gene that results in more efficient expression. Upstream, as used herein, is defined as a nucleotide sequence which is transcribed and translated before the aFGF nucleotide sequence. Expression vector as used herein is defined as any autonomously replicating agent, including but not limited to plasmids, comprising a DNA molecule to which one or more transcriptional and translational activator sequence or sequences have been incorporated. Expression vectors which can be used to produce enhanced expression vectors include, but are not limited to, pPLa2311, pKC30,ptac 12, lambda gt 11, pAs1, PLC24, pSB226, pRIT2T, SV40, pBR322 and pKK223-3 with pKK223-3 being preferred. The expression vector and the procedure for incorporating the synthetic gene for the 140 amino acid form of haFGF is described by Linemeyer et al., European Patent Application, Publication No. 259,953. It is to be understood that the procedures used for the expression of the 140 amino acid form of haFGF can be used for the expression of the other microheterogeneous forms of haFGF.

Enhanced expression vectors will contain a first cistron or enhancing cistron which is responsible for the enhanced expression and a second cistron which encodes for haFGF. Cistron is defined as a region of DNA that codes for a particular peptide or may simply function as a specific sequence of nucleotides which are not translated but can enhance the expression of closely associated cistrons. The enhancing cistron will contain a translational initiation codon (ATG) followed by a Shine-Dalgarno sequence with a nucleotide base complementary to the 16S ribosomal RNA (rRNA) binding site consisting of about 8 bases and followed by a stop codon (TAA). The enhancing cistron, which is upstream of the haFGF cistron, will be operably attached to the haFGF cistron and will be downstream of the promoter. Operably attached refers to an appropriate sequential arrangement of nucleotide segments, cistrons or genes, such that the desired protein will be produced by cells containing an expression vector containing the operably attached genes or cistrons and an appropriate promoter and termination system. The efficiency of the translation of the second cistron coding for haFGF is determined by the length of the enhancing cistron, location of the Shine-Dalgarno sequence in relation to the stop codon in the first cistron and the location of the stop codon in the first cistron in relation to the start codon of the haFGF cistron. The enhancing first codon will be from about 6 to about 12 codons in length. The preferred location for the stop codon in the first cistron is downstream from the Shine-Dalgarno sequence and within about 2 bases of the start codon of the second cistron. Enhancing cistrons include, but are not limited to, the nucleotide base sequences in the following table which are similar to those described by Schoner et al., Proc. Natl. Acad. Sci. USA 83: 8506-8510 (1986).

## TABLE 1

ATG TAT CGC GAT TTA AAT AAG GAG GAA TAA

ATG TAT CGA TTA AAT AAG GAG GAA TAA

ATG TAT CGT GAA TTC CGA TTA AAT AAG GAG GAA TAA

The nucleotide sequence of the preferred enhancing cistron is shown in the following table.

## TABLE 2

5' AATTATGTATCGATTAAATAAGGAGGAAT 3'
3' TACATAGCTAATTTATTCCTCCTTATTAA 5'
(plasmid)   (cistron 1, 2 oligomers)   (haFGF)

The first cistron is inserted into the appropriate pKK-haFGF construct at the EcoRl site as described in Linemeyer et al., European Patent Application Publication No. 259,953. The insertion results in the loss of the EcoRl cloning site. The recombinant is transformed into an appropriate host cell such as E. coli JM105 (Pharmacia) or DH5 (BRL) and expressed. The plasmids containing the enhancing expression vector are designated pKK2c-haFGF. The present invention is contemplated to include clones containing the enhancing expression vector such as pKK2c-haFGF (154), pKK2c-haFGF (140) and pKK2c (139). Expression is carried out under conditions known in the art, such as those described in Linemeyer et al., Bio/Tech. 5: 960-965 (1987).

Cells expressing r-haFGF, single and double cistron expression systems, are suspended in a buffer containing protease inhibitors and disrupted. The cell free lysate is mixed with a phosphate buffer, pH 6.0, an ion exchange resin, CM-Sephadex, loaded in a column and eluted with 0.60 M NaCl in a phosphate buffer. The positive fractions are collected, pooled and the pH adjusted to 7.2. The samples are added to Heparin-Sepharose and added to a column. The r-haFGF is eluted with 10 mM sodium phosphate, 1.5 M NaCl at a pH of 7.2. The fraction containing r-haFGF is further purified by reversed-phase HPLC as described by Thomas et al., Proc. Natl. Acad. Sci. USA 81: 357-361 (1984). Polyacrylamide gel electrophoresis is used to confirm purity while identity of r-haFGF is confirmed by amino acid analysis and amino terminal sequence determination. When r-haFGF produced by the single and double cistronic expression systems are compared by Western Immunoblot analysis using te method of Linemeyer et al., Bio/Tech. 5: 960-965 (1987), the double cistron system expresses r-haFGF at a level about 10-fold higher than the single cistron system. The concentration of r-haFGF is determined using an extinction coefficient process as described by Thomas, Methods Enzymol. 147: 120-135 (1987) and an analytical HPLC method as described in Example 3. When amounts of single and double cistron expressed r-haFGF are compared by the extinction coefficient process the single cistron expression system produced about 2.2 mg of highly purified r-haFGF per 1 x $10^{12}$ transformed cells while the double cistron expression system produced about 29.2 mg of r-haFGF for the same cell concentration. The double cistron system increased the amount of r-haFGF by about 13.3-fold. The analytical HPLC method for determining r-haFGF concentration revealed that there was about a 8.2-fold increase in the clarified lysate, about a 6.0-fold increase following CM-Sepharose chromatography and about a 5.4-fold increase after Heparin-Sepharose chromatography.

The ability of aFGF and r-haFGF to stimulate division in various cell types including fibroblasts, vascular and corneal endothelial cells and the like makes r-haFGFs useful as a pharmaceutical agent. Recombinant haFGF is useful in promoting the healing or repair of, but not limited to, soft tissue wounds resulting from burns, cuts or lacerations, and cuteaneous ulcerations along with musculo-skeletal wounds such as bone fractures, ligament and tendon tears, and inflammation of bursas and tendons. Tissue repair as used herein is defined as the regeneration of tissue following the stimulation of cells by r-haFGF. Recombinant haFGF is also useful in promoting the healing and regeneration of cartilage and cartilaginous tissue. Pharmaceutical preparations of r-haFGF for use in tissue repair or wound healing will generally contain a sulfated glycosaminoglycan such as heparin.

The following examples illustrates the present invention without, however, limiting the same thereto.

## EXAMPLE 1

### Enhanced Expression Vector

Enhanced levels of expression for haFGF were obtained by the modification of the expression vector PKK-haFGF, using procedures in Linemeyer et al., Bio/Tech. 5: 960-965 (1987), to introduce an additional cistron upstream of the haFGF encoding sequence. The preferred first cistron was synthesized as two oligomers with the sequences as shown in Table 2. The oligomers are synthesized by methods well known in the art, such as those described by Matteucci and Caruthers, J. Am. Chem. Soc. 103: 3185-3191 (1981); Beaucage and Caruthers, Tetrahedron Letters 22: 1859-1862 (1981). When annealed these oligomers supply 5' extensions of 4 bases which are complementary to the extensions provided by EcoRI cleavage, a 7 codon open reading frame following the ATG translation initiation codon and preceding a TAA stop codon, and an additional Shine-Dalgarno ribosome binding site located within the open reading frame upstream of the stop codon. Using 1 pmole of each oligomer, the oligomers were annealed together in 20 μl of DNA ligase buffer by heating to 70° C for 10 minutes and slow cooling. The annealed mixture, 0.3 pmole, was ligated to 0.1 pmole of EcoRI-cleaved PKK-haFGF plasmid DNA, see Fig. 1, in a final volume of 25 μl containing 3 units of T4 DNA ligase (Pharmacia) for 2.5 hours at 14° C. The ligated DNA, 5 ng, was used to transform competent E. coli JM105 cells by standard procedures known in the art. The transformants were screened by restriction analysis, as the EcoRI site is lost by this insertion, and by immunoblot analysis. The expression vector of a clone, which demonstrated higher levels of haFGF production, was sequenced by the chemical technique of Maxam and Gilbert, Proc. Natl. Acad. Sci. USA 74: 560-564 (1977), to verify the correct insertion of the new cistron sequence.

The enhanced expression pKK2c-haFGF vector was lotransferred to E. coli DH5 by standard transformation procedures known in the art.

The expression clones were grown at 37°C in LB broth (1% tryptone, 0.5% yeast extract, 0.5% NaCl) containing 0.4% glucose and 50 $\mu$g/ml ampicillin. When the optical density at 550 nm reached 0.5, IPTG was added to give 1 mM and growth was continued at 37°C for 3 hours. The cells were harvested by centrifugation at 10,000 x g for 20 minutes and the cells from 1 liter of culture were resuspended in 20 ml of 10mM sodium phosphate pH 7.2, (heparin-Sepharose buffer) 5 mM EDTA, 10.6 $\mu$g/ml TPCK, 34.3 $\mu$g/ml pepstatin A, 87 $\mu$g/ml PMSF, 15 $\mu$g/ml BPTI, and 34.3 $\mu$g/ml leupeptin. The resuspended cells were quickly frozen in a dry ice/ethanol bath and stored overnight at -70°C.

## EXAMPLE 2

### Extraction and Purification of Recombinant haFGF

The frozen cells from Example 1 were thawed, mixed with an equal volume of 100 mM sodium phosphate, pH 6.0, containing 5 mg/ml EDTA and centrifuged at 27,000 x g for 5 minutes at 4°C. The cells were washed and resuspended in 60 ml of the phosphate buffer containing; 0.05 mM PMSF, 0.03 mM TPCK, 0.05 mM pepstatin A, 0.05 leupeptin and 15$\mu$g/ml BPTI prior to disruption. The cell suspension was passed through a French pressure cell at 12,000 psi three times with a constant temperature of 4°C. The resulting lysate was centrifuged at 27,000 x g for 15 minutes at 4°C to remove cell debris and the supernatant fluid from the final centrifugation was collected, quickly frozen in a dry ice/ethanol bath and stored at -70°C.

The cell-free frozen lysate was combined with 200ml of 100 mM sodium phosphate, pH 6.0, and washed CM-Sephadex at a rate of 6.5 ml/gm protein. The suspension was mixed and washed in a sintered glass funnel with three 200 ml washes of 100mM phosphate, pH 6.0, containing 0.15 M NaCl. The CM-Sephadex-lysate complex was packed into a 2.5 cm diameter column and eluted at a rate of 12 ml/hr/cm$^2$ with an eluant consisting of 100 mM phosphate, pH 6.0, and 0.6 M NaCl. Fractions were monitored by absorbance at 280 nm and those containing protein were collected, pooled and diluted with cold deionized water to about 10 $\mu$S/cm conductivity. The protein sample was adjusted to pH 7.2 with 1 N NaOH and then added to Heparin-Sepharose which had been washed with 10 mM phosphate, pH 7.2 containing 2 M NaCl and equilibrated in 10 mM phosphate, pH 7.2. The suspension containing 1 ml packed Heparin-Sepharose per mg protein was gently stirred for 1 hr at 4°C, collected on sintered glass, packed into a 1 cm diameter column and eluted with a 2 column volume/hour flow rate. After loading, the column was washed with 10 mM sodium phosphate, 0.8 M NaCl, ph 7.2 until the absorbance at 280 nm fell to background. Bound r-haFGF was eluted as a single peak with 10 mM sodium phosphate, 1.5 M NaCl, pH 7.2. The pooled fractions from the heparin-Sepharose column were purified by reversed-phase HPLC using a 4.6 mm x 25 cm C$_4$ column (Separations Group) as described by Thomas et al., Proc. Natl. Acad. Sci. USA 81: 357-361 (1984). The r-haFGF eluted as a single major peak that was resolved from multiple minor contaminant peaks suggesting that the protein was homogeneously pure. Polyacrylamide gel electrophoresis was used to confirm purity. The purifiedr-haFGF was reduced and electrophoresed in the presence of sodium dodecyl sulfate through a 15% polyacrylamide gel prepared as described by O'Farrell, J. Biol. Chem. 250: 4007-4021 (1975). Silver staining revealed a single band with a molecular mass of 16,000 daltons. Identity of the protein as haFGF was confirmed by both amino acid analysis and amino terminal sequence determination.

The amount of pure r-haFGF recovered was determined using the extinction coefficient at 280 as described by Thomas, Methods Enzymol. 147: 120-135 (1987). The single cistron expression system produced 2.2 mg of highly purified r-haFGF/1 x 10$^{12}$ transformed cells while the double cistron expression produced 29.2 mg of highly purified r-haFGF/1 x 10$^{12}$ transformed cells. The double cistron system resulted in a 13.3-fold increase in r-haFGF expression.

## EXAMPLE 3

Comparison Of Acidic Fibroblast Growth Factor Production By Single And Double Cistronic Expression Systems In Large Batch Fermentations

Escherchia coli containing the single cistron expression vector, pKK-haFGF, and the double cistron expression vectors, such as pKK2c-haFGF, from Example 1, were grown and purified under identical scale-up conditions. These conditions allowed a direct comparison of aFGF production under the two expression systems. Expression clones were grown in a buffered growth medium (0.1% yeast extract, 0.5% HySoy and normal salts, pH 7.0) containing 50 μg/ml ampicillin in a fermentor operated to maintain pH and dissolved oxygen of the broth. When the absorbance at 550 nm reached 0.5, 1 mM IPTG was added and growth was continued at 37°C for seventeen hours or until maximal aFGF was expressed. Two 9.5 L batches of each transformed cell type were pooled washed and concentrated in preparation for disruption. In the presence of protease inhibitors, Example 2, the washed cells were disrupted with a Manton-Gaulin Laboratory Homogenizer. The single and double cistron expressed r-haFGFs were purified by the multiple step chromatographic process of Example 2.

Concentrations of aFGF in the various samples were determined by analytical HPLC using a $C_4$ column (Vydac). The column was equilibrated in solvent A (10 nM trifluoroacetic acid in water). The flow rate was 1.5 ml/minute. Acidic FGF elutes when the percent of solvent A is between 60% and 40% and the percent of solvent B (67% acetonitrile/33% solvent A) is between 40% and 60%, approximately 35% acetonitrile. Under these conditions aFGF preparative column eluants have been shown to yield a linear response on HPLC in concentrations up to 0.89 mg/ml. Therefore, samples with higher concentrations of aFGF require dilution for accurate quantification. All dilutions are made with the eluting buffer. Standards are prepared and assayed as described and product concentration is calculated by dividing the sample peak area by the standard peak area and multipling by the standard concentration. The following table compares aFGF concentration for single and double cistron expression systems. Equal volumes of fermentation broth were processed for the single and double cistron systems. Cell concentrations were adjusted to a constant value prior to homogenization. Subsequent loadings of chromatographic resins were based upon total protein content of the samples. The total aFGF values of the single cistron system (2.7 1 lysate processed) are normalized to the double cistron system (1.455 1 processed).

TABLE 1

| Comparison Of Acidic Fibroblast Growth Factor Production By Single And Double Cistronic Expression Systems | | | |
|---|---|---|---|
| Cistron | Process Step | aFGF Concentration mg/ml | Total aFGF mg |
| Single | Clarified Lysate | 0.074 | 108 |
| | CM Sephadex | 0.197 | 89 |
| | Heparin Sepharose Eluate | 0.237 | 64 |
| Double | Clarified Lysate | 0.311 | 889 |
| | CM Sephadex Eluate | 2.97 | 533 |
| | Heparin Sepharose Eluate (1) | 1.891 | 244 |
| | Heparin Sepharose Eluate (2) | 0.887 | 103 |
| Total Heparin Sepharose Eluate | | | 347 |

A 4-8 fold increase in aFGF productivity was observed in recombinant E. coli employing the double cistron expression system when compared to expression levels for the single cistron system. The level was highest in the clarified lysate and decreased to about 4-5-fold as the samples were purified.

**Claims**

1. A process for enhancing the expression of human acidic fibroblast growth factor expressed in transformed procaryotic cells which comprises:

a. providing an expression vector which vector comprises:

i. a first cistron, which contains a translational initiation codon, a Shine-Dalgarno sequence and a stop sequence, operably linked to and upstream of the second cistron with both cistrons being under control of the same promoter;

ii. a second cistron which encodes acidic fibroblast growth factor; and

b. transforming a procaryotic cell with the vector described in a; and

c. culturing the transformed cell under growth conditions suitable for gene expression and production of acidic fibroblast growth factor.

2. The process of Claim 1, step a, section ii wherein the Shine-Delgarno sequence is about eight nucleotides in length.

3. The process of Claim 1, step b wherein the procaryotic cell is Escherichia coli.

4. The process of Claim 1, step a, section i wherein the first cistron is selected from the group consisting of:

ATG TAT CGC GAT TTA AAT AAG GAG GAA TAA

ATG TAT CGA TTA AAT AAG GAG GAA TAA

ATG TAT CGT GAA TTC CGA TTA AAT AAG GAG GAA TAA.

5. The first cistron of Claim 4 wherein the cistron has the nucleotide base sequence of:
ATG TAT CGA TTA AAT AAG GAG GAA TAA.

6. The process of Claim 1, step a, section ii wherein the human acidic fibroblast growth factor cistron encodes for the 154 amino acid residue microheterogeneous form of human acidic fibroblast growth factor and having an additional codon coding for a methionine residue with said additional codon being attached to the ordinarily first codon at the N-terminus of said microheterogeneous form.

7. The process of Claim 1, step a, section ii wherein the human acidic fibroblast growth factor cistron encodes for the 140 amino acid residue microheterogeneous form of human acidic fibroblast growth factor and having an additional codon coding for a methionine residue with said additional codon being attached to the ordinarily first codon at the N-terminus of said microheterogeneous form.

8. The process of Claim 1, step a, section ii wherein the human acidic fibroblast growth factor cistron encodes for the 139 amino acid residue microheterogeneous form of human acidic fibroblast growth factor and having an additional codon coding for a methionine residue with said additional codon being attached to the ordinarily first codon at the N-terminus of said microheterogeneous form.

9. The process of Claim 8 wherein the amino terminal asparagine amino acid residue of the 139 amino acid microheterogeneous form of human acidic fibroblast growth factor is deamidated to aspartic acid.

10. The process of Claim 1 wherein there is about a 5 to 13-fold increase in aFGF production compared to a single cistron expression system.

11. Acidic fibroblast growth factor characterized by having been prepared by the process of Claim 1.

12. A strain of Escherichia coli transformed with the double cistron expression system of Claim 1 wherein the levels of expression of acidic fibroblast growth factor is increased by at least 5-fold.

13. A double cistron expression system for the enhanced expression of human acidic fibroblast growth factor comprising an enhancing cistron operably attached to a cistron encoding human acidic fibroblast growth factor, with the enchancing cistron containing a translation codon, a Shine-Dalgarno sequence and a stop codon and with both cistrons being under control of the same promoter.

# FIG-1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | PROC. NATL. ACAD. SCI. USA, vol. 83, November 1986, pages 8506-8510; B.E. SCHONER et al.: "Translation of a synthetic two-cistron mRNA in Escherichia coli" * The whole document; especially page 8508, figure 4 * | 1-13 | C 12 N 15/16 C 12 P 21/02 C 12 N 15/70 |
| D,Y | EP-A-0 259 953 (MERCK & CO., INC.) * Page 4, line 18 - page 5, line 5; page 20, line 21 - page 21, line 13; claims 27-51 * | 1-13 | |
| P,X | EP-A-0 319 052 (MERCK & CO. INC.) * Page 19, lines 29-48; page 26, example 6; claim 12 * | 1-5,10-15 | |

TECHNICAL FIELDS
SEARCHED (Int. Cl.5)

C 12 N
C 12 P

The present search report has been drawn up for all claims

| Place of search | Date of completion of the search | Examiner |
|---|---|---|
| THE HAGUE | 05-07-1990 | ANDRES S.M. |

EPO FORM 1503 03.82 (P0401)